# EUROPÄISCHE PATENTANMELDUNG

(11) **EP 1 027 867 A1**
(43) Veröffentlichungstag der Anmeldung: **16.08.2000**
(21) Anmeldenummer: 00810103.2
(22) Anmeldetag: 07.02.2000
(51) Int. Cl.: A61B 17/34, A61M 25/02

(54) **Vorrichtung zum Halten einer Hülse und Verfahren zum Herstellen einer solchen Vorrichtung**

(30) Priorität: 09.02.1999 CH 24399
(71) Anmelder: Berger AG, 3645 Gwatt/Thun (CH)
(72) Erfinder: Berger, Fritz, 3600 Thun (CH)
(74) Vertreter: Wenger, René

(57) **Zusammenfassung**

Eine Vorrichtung zum Halten einer die Oberfläche eines menschlichen oder tierischen Körpers durchdringenden Hülse auf der Oberfläche besteht im wesentlichen aus zwei Abschnitten (4a, 4b). Die beiden Abschnitte sind über eine federnde Verbindung (5) miteinander verbunden und je mit einer eine Längsrichtung (La, Lb) definierenden Längsöffnung (3a, 3b) versehen. In einer Klemmlage (K) verlaufen die Längsrichtungen (La, Lb) der Längsöffnungen (3a, 3b) der Abschnitte (4a, 4b) unter einem Winkel (α) zueinander. In einer Verschiebelage (V) verlaufen die Längsrichtungen (La, Lb) etwa parallel zueinander. Eine in die Längsöffnung (3) eingesetzte Hülse (2) lässt sich in der Längsöffnung (3) verschieben, wenn die Haltevorrichtung sich in der Verschiebelage (V) befindet. Wenn die Haltevorrichtung sich in der Klemmlage (K) befindet, ist die Hülse nicht verschiebbar eingeklemmt.

## Beschreibung

Vorrichtung zum Halten einer Hülse und Verfahren zum Herstellen einer solchen Vorrichtung

Die Erfindung betrifft eine Vorrichtung zum Halten einer die Oberfläche eines menschlichen oder tierischen Körpers durchdringenden Hülse auf dieser Oberfläche und ein Verfahren zum Herstellen einer solchen Vorrichtung mit den Merkmalen des Oberbegriffs der unabhängigen Patentansprüche.

Bei der Durchführung von chirurgischen Eingriffen werden häufig zur Unterstützung des chirurgischen Eingriffs oder zu diagnostischen Zwecken Endoskope eingesetzt. Dabei wird die Oberfläche des menschlichen oder tierischen Körpers mit einer Hülse durchdrungen. Die Hülse dient zum Einführen von Sonden, chirurgischen Hilfsinstrumenten oder eines Gases (Insufflation) in eine Körperhöhle. Typischerweise durchdringt die Hülse die Bauchdecke, um Hilfsmittel oder Gase in den Bauchraum einzuführen.

Es sind Vorrichtungen bekannt, die dazu dienen, die die Oberfläche durchdringende Hülse auf der Haut zu befestigen. Solche bekannten Vorrichtungen weisen eine Durchtrittsöffnung für die Hülse auf, in welcher die Hülse verschiebbar einsetzbar ist. Diese Haltevorrichtungen dienen dazu, die Hülse in einer bestimmten Lage zu halten und auch dazu, die Durchdringöffnung in der Oberfläche des Körpers abzuschliessen.

Üblicherweise wird die Haltevorrichtung mittels eines in der Bauchdecke angebrachten Fadens auf der Oberfläche des Körpers befestigt. Damit die Hülse in einer bestimmten Stellung gegenüber der Bauchdecke festgehalten werden kann, sind die bekannten Haltevorrichtungen mit einem Klemmmechanismus versehen. Der Klemmmechanismus erlaubt es, die Hülse in der Durchtrittsöffnung der Haltevorrichtung zu verschieben und in einer gewünschten Lage festzustellen. Der Klemmmechanismus bei bekannten Vorrichtungen besteht aus mehreren miteinander verbundenen Komponenten. Aufgrund der Verbindungen (welche oft als gelenkige Verbindungen ausgebildet sind) ist die Reinigung und Sterilisation solcher Haltevorrichtungen nicht oder nur schwierig möglich.

Aufgrund der mehrteiligen Konstruktion ist auch die Herstellung der bekannten Haltevorrichtungen aufwendig und diese sind deshalb teuer.

Es ist eine Aufgabe der vorliegenden Erfindung, die Nachteile des Bekannten zu vermeiden, insbesondere eine Vorrichtung zum Halten einer Hülse zu schaffen, welche mehrfach verwendbar ist, welche auf einfache Weise herstellbar ist und welche zur Reinigung und Sterilisation geeignet ist. Eine weitere Aufgabe der vorliegenden Erfindung besteht darin, ein Verfahren zum Herstellen einer solchen Haltevorrichtung zu schaffen, welches einfach und auf wirtschaftliche Weise durchführbar ist.

Erfindungsgemäss werden diese Aufgaben mit einer Vorrichtung und einem Verfahren mit den Merkmalen des kennzeichnenden Teils der unabhängigen Patentansprüche gelöst.

Die Vorrichtung weist wenigstens zwei Abschnitte auf, die mit einer federnden Verbindung miteinander verbunden sind. In jedem der Abschnitte ist ein Teil der in einer Längsrichtung verlaufenden Längsöffnung angebracht. Die federnde Verbindung ist dabei derart gewählt, dass sich die Abschnitte in einer Klemmlage an einer eingesetzten Hülse verspannen und dass die Abschnitte gegen die Federkraft in eine Verschiebelage bringbar sind, in der die Hülse durch die Längsöffnung verschiebbar ist.

In einem bevorzugten Ausführungsbeispiel ist in einer Klemmlage die gegenseitige Lage der Abschnitte derart gewählt, dass die Längsrichtungen der Längsöffnungen der beiden Abschnitte in einem Winkel zueinander verlaufen. In einer Verschiebelage verlaufen die Längsrichtungen der Längsöffnungen der beiden Abschnitte etwa parallel zueinander.

Weil in der Verschiebelage die Längsrichtungen der beiden Längsöffnungen etwa parallel zueinander verlaufen, kann eine in die Längsöffnungen eingesetzte Hülse einfach verschoben werden.

Weil die Längsrichtungen in der Klemmlage im Winkel zueinander stehen, verklemmt sich eine in die Längsöffnungen eingesetzte Hülse. Aufgrund dieser Klemmwirkung ist die Hülse in der Haltevorrichtung nicht mehr verschiebbar.

Die Längsöffnungen in den Abschnitten sind vorzugsweise zylindrisch ausgebildet, da Hülsen für die Endoskopie üblicherweise ebenfalls zylindrisch ausgebildet sind.

Wenn der Aussendurchmesser der Hülse geringer ist als der freie Innendurchmesser der Längsöffnungen, ist die Hülse in der Haltevorrichtung selbst dann noch verschiebbar, wenn die Längsrichtungen der beiden Längsöffnungen in einem kleinen Winkel zueinander stehen. Sobald dieser Winkel allerdings zu gross wird, ergibt sich auch bei einer solchen Dimensionierung eine Klemmwirkung.

Bevorzugt ist allerdings der freie Innendurchmesser der Längsöffnungen gleich gross wie der Aussendurchmesser der Hülse gewählt.

In einem besonders bevorzugten Ausführungsbeispiel ist in der Klemmlage die federnde Verbindung im wesentlichen entspannt und in der Verschiebelage ist die federnde Verbindung gespannt. Insbesondere sind in der Verschiebelage die beiden Abschnitte vorteilhaft gegeneinander gedrückt. Es ist aber auch denkbar, dass zum Erreichen der Verschiebelage die Abschnitte gegen die federnde Verbindung auseinander gezogen werden.

Die federnde Verbindung ist dabei in der Klemmlage nicht vollständig entspannt. Wichtig ist in diesem Ausführungsbeispiel, dass die Vorrichtung in Abwesenheit von äusserer Krafteinwirkung selbstätig die Klemmlage einnimmt. Der Vorteil dieses Ausführungsbeispiels besteht darin, dass zum Verschieben der Hülse die beiden Abschnitte gegeneinander gespannt, beispielsweise gegeneinandergedrückt werden müssen, und dass ohne Zutun automatisch immer die Klemmlage eingenommen wird.

Vorteilhaft ist die Vorrichtung einstückig, beispielsweise aus einem sterilisierbaren Kunststoffmaterial ausgebildet. Dies ist im Hinblick auf eine einfache Herstellung und eine einfache Sterilisierbarkeit besonders günstig. Es wäre aber auch denkbar, eine Haltevorrichtung vorzusehen, welche aus zwei gelenkig miteinander verbundenen Abschnitten besteht, die mit einer eingesetzten Feder gegeneinander gespannt werden. Um Sterilisierbarkeit zu gewährleisten, reicht es aus, die einzelnen Komponenten leicht lösbar voneinander auszubilden.

Es ist auch denkbar, Abschnitte vorzusehen, die seitlich zueinander verschiebbar sind und bei denen in der Verschiebelage die Öffnungen fluchten und in der Klemmlage versetzt sind.

Es ist ausserdem auch denkbar, die Haltevorrichtung aus Metall, beispielsweise Edelstahl auszubilden.

Bei einer einstückig ausgebildeten Haltevorrichtung kann die federnde Verbindung durch ein einstückig mit den beiden Abschnitten ausgebildetes Biegegelenk besonders einfach geformt werden. Die Abschnitte können durch einen etwa senkrecht zur Längsrichtung verlaufenden Spalt voneinander getrennt sein.

Zur Erleichterung der Handhabung der Haltevorrichtung ist es ausserdem vorteilhaft, die Abschnitte mit vorstehenden Grifflaschen zu versehen, mittels welchen die Abschnitte gegeneinander zusammengedrückt oder gegebenenfalls auch auseinandergezogen werden können.

Die Vorrichtung kann ausserdem in bekannter Weise mit einem etwa konischen Dichtteil versehen sein, der zum Verschliessen der Durchtrittsöffnung in der Oberfläche des Körpers dient. Ausserdem kann die Vorrichtung Haltevorsprünge aufweisen, die zum Befestigen eines in die Haut eingenähten Fadens dienen.

Anstelle von Hülsen im eigentlichen Sinn können selbstverständlich mit der erfindungsgemässen Halterung auch andere medizinische Geräte wie optische Leiter, Schläuche o.ä. gehalten werden, die die Oberfläche eines Körpers durchdringen.

Das Verfahren zum Herstellen der vorangehend beschriebenen Vorrichtung besteht im wesentlichen darin, dass zuerst ein Rohling bereitgestellt wird und dass anschliessend in den Rohling die Längsöffnungen angebracht werden.

Erfindungsgemäss wird ein Rohling bereitgestellt, der zwei federnd miteinander verbundene Abschnitte aufweist. Vor dem Anbringen der Längsöffnungen werden die Abschnitte gegen die federnde Verbindung in eine Verschiebelage gebracht, typischerweise zusammengedrückt. Während sich die Abschnitte in Verschiebelage befinden, wird eine die beiden Abschnitte durchdringende Längsöffnung, insbesondere eine Bohrung angebracht. Mit diesem Herstellungsverfahren ist sichergestellt, dass in der Verschiebelage die Längsrichtungen der Längsöffnungen in den beiden Abschnitten parallel zueinander verlaufen.

Die beiden Abschnitte können beispielsweise dadurch voneinander getrennt werden, dass im Rohling ein Spalt angebracht, beispielsweise eingefräst wird.

Vorzugsweise werden die beiden Abschnitte nach dem Anbringen der durchgehenden Längsöffnung (z.B. nach dem Entfernen eines Bohrers) weiter in der Verschiebelage gehalten. Anschliessend wird eine Hülse in die Längsöffnung eingesetzt. Dies ist besonders einfach möglich, weil sich die Haltevorrichtung noch in der Verschiebelage befindet. Sobald die Abschnitte nicht mehr in der Verschiebelage gehalten werden, stellen sie sich aufgrund der federnden Verbindung in eine, die Klemmlage definierende Position zurück und fixieren die zuvor eingesetzte Hülse.

Die Erfindung wird im folgenden anhand der Zeichnungen näher erläutert. Es zeigen:
- Figur 1: eine schematische Darstellung einer Hülse und einer Haltevorrichtung in der Seitenansicht,
- Figur 2: einen Querschnitt durch eine Haltevorrichtung in Verschiebelage,
- Figur 3: einen Querschnitt durch die Haltevorrichtung in Klemmlage, und
- Figur 4: eine Draufsicht auf die erfindungsgemässe Haltevorrichtung.

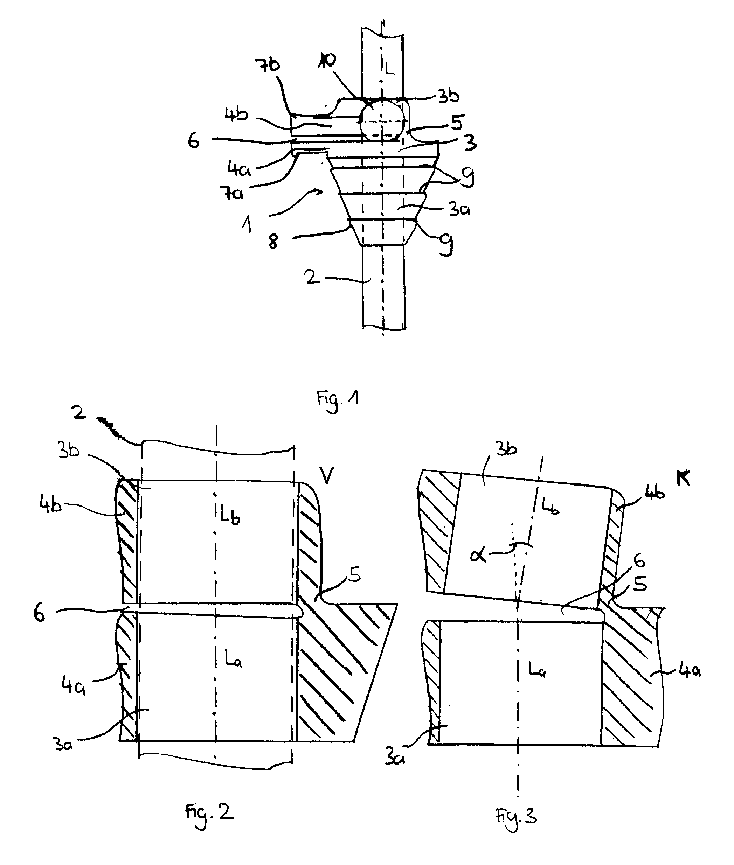

Figur 1 zeigt eine Haltevorrichtung 1, die mit einer durchgehenden Längsöffnung 3 versehen ist. In die durchgehende Längsöffnung 3 ist eine Hülse 2 eingesetzt. Die Hülse 2 besteht beispielsweise aus Edelstahl.

Die Vorrichtung 1 besteht im wesentlichen aus einem unteren Abschnitt 4a und einem oberen Abschnitt 4b. Die Abschnitte 4a, 4b sind über ein Biegegelenk 5 miteinander federnd verbunden.

Der untere Abschnitt 4a ist mit einer ersten Längsöffnung 3a und der obere Abschnitt 4b mit einer zweiten Längsöffnung 3b versehen. Die Längsöffnungen 3a, 3b bilden zusammen die durchgehende Längsöffnung 3 durch die Haltevorrichtung 1.

Die beiden Abschnitte 4a, 4b sind durch einen etwa senkrecht zur Längsrichtung L der Längsöffnung 3 verlaufenden Spalt 6 voneinander getrennt. Aufgrund des Spaltes 6 können die beiden Abschnitte 4a, 4b gegen die durch das Biegegelenk 5 aufgebrachte Federkraft zusammengedrückt werden. Zum Zusammendrücken der Abschnitte 4a, 4b sind diese mit Grifflaschen 7a, 7b versehen, welche seitlich aussen vorstehen.

Die Haltevorrichtung 1 ist ausserdem mit einer etwa konischen Dichtfläche 8 versehen. Die Dichtfläche 8 kann ausserdem mit Rippen 9 versehen sein. Die Dichtfläche 8 dient zum Verschliessen der Durchdringöffnung in der Oberfläche des Körpers, durch welche die Hülse 2 in eine Körperhöhle eindringt.

Ausserdem sind an der Haltevorrichtung 1 im oberen Abschnitt 3b Haltevorsprünge 10 vorgesehen, um welche ein in die Haut genähter Faden zum Halten der Haltevorrichtung auf der Oberfläche des Körpers gewickelt werden kann.

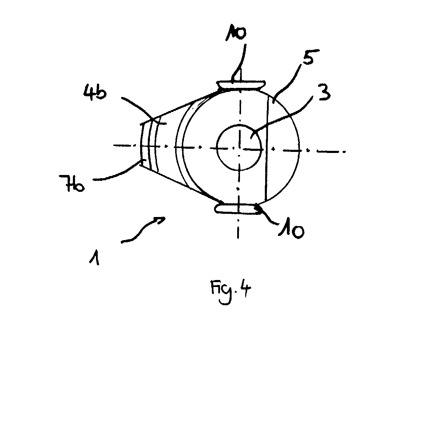

Aufgrund der federnden Verbindung tendieren die beiden Abschnitte 4a, 4b dazu, eine entspannte Lage einzunehmen. Diese entspannte Lage definiert eine Klemmlage K (siehe Figur 3), in welcher die Längsrichtungen 3a, 3b der Abschnitte 4a, 4b im Winkel zueinander stehen. Eine in die Längsöffnung 3 eingeführte Hülse 2 wird aufgrund der im Winkel zueinander stehenden Längsöffnungen 3a, 3b verklemmt und lässt sich nicht mehr verschieben. Durch Zusammendrücken der Abschnitte 4a, 4b im Bereich der Grifflaschen 7a, 7b werden die Längsrichtungen La, Lb der Längsöffnungen 3a, 3b zueinander ausgerichtet. Die zueinander ausgerichteten Längsrichtungen La, Lb definieren eine Verschiebelage V, in welcher die Hülse 2 sich in der Haltevorrichtung 1 verschieben lässt.

Figur 2 zeigt einen Querschnitt durch die Haltevorrichtung 1 in der Verschiebelage V.

Die beiden Abschnitte 4a, 4b sind gegen die Federkraft des Biegegelenkes 5 zusammengedrückt. Die Längsrichtung La der Längsöffnung 3a des unteren Abschnitts 4a verläuft in der Verschiebelage V parallel zur Längsrichtung Lb der Längsöffnung 3b des oberen Abschnittes 4b. Die Hülse 2 lässt sich deshalb einfach in der Haltevorrichtung 1 verschieben.

In Figur 3 ist ein Querschnitt durch die Haltevorrichtung in einer Klemmlage K gezeigt. In der Klemmlage ist die durch das Biegegelenk 5 erzeugte federnde Verbindung zwischen dem unteren Abschnitt 4a und dem oberen Abschnitt 4b etwa entspannt. Die Längsachse La der Längsöffnung 3a des unteren Abschnitts 4a verläuft unter einem Winkel α zur Längsrichtung Lb der Längsöffnung 3b des oberen Abschnitts 4b. In Figur 3 ist der Winkel α aus darstellerischen Gründen stark übertrieben dargestellt. Der Winkel a beträgt in Realität etwa 3 bis 6 Grad.

Aufgrund der in unterschiedlichen Richtungen verlaufenden Längsöffnungen 3a, 3b verklemmt sich eine in die durchgehende Längsöffnung 3 eingesetzte Hülse 2. Wegen der federnden Verbindung zwischen den Abschnitten 4a, 4b verbleiben diese ohne äussere Krafteinwirkung in der Klemmlage. Die Hülse kann erst verschoben werden, wenn die beiden Abschnitte 4a, 4b gegen die Federkraft in die in Figur 2 gezeigte Verschiebelage gebracht werden.

In Figur 4 ist eine Draufsicht auf die Haltevorrichtung 1 gezeigt.

Seitlich an der Haltevorrichtung 1 sind Haltevorsprünge 10 vorgesehen, um welche ein in die Haut eingenähter Faden zum Befestigen der Haltevorrichtung gewickelt werden kann.

Die Längsöffnung 3 ist als Bohrung ausgebildet, welche zur Aufnahme einer gebräuchlichen Hülse dient. Die Bohrung hat deshalb einen Durchmesser von etwa 10 - 15 mm.

Seitlich vorstehend sind Grifflaschen 7b und 7a (in Figur 4 versteckt) vorgesehen, welche zum Zusammendrücken der Abschnitte 4a, 4b gegeneinander dienen.

Die Haltevorrichtung 1 wird typischerweise aus einem Rohling aus einem sterilisierbaren Kunststoffmaterial, wie Ertacetal etc. hergestellt. In den Rohling wird zuerst ein Spalt 6 (siehe Figuren 1 bis 3) eingefräst, der sich im wesentlichen quer durch die Haltevorrichtung erstreckt, so dass zwei durch ein Biegegelenk 5 gebildete Abschnitte 4a, 4b gebildet werden. Vor dem Anbringen einer durchgehenden Längsbohrung 3 werden die Abschnitte 4a, 4b gegen die Federkraft des Biegegelenkes 5 in die Verschiebelage V zusammengedrückt. Die durchgehende Längsöffnung 3 wird in der Verschiebelage V angebracht, typischerweise gebohrt. Nach Entfernen des Bohrers wird die Haltevorrichtung in der Verschiebelage belassen und die Hülse 2 wird eingesetzt. Nach Einsetzen können die Abschnitte 4a, 4b losgelassen werden, so dass sie sich aufgrund der federnden Verbindung so weit möglich in die ursprüngliche Lage zurücksetzen. Ein vollständiges Zurücksetzen in die ursprüngliche Lage kann aufgrund der eingesetzten Hülse 2 nicht möglich sein. Die nach dem Loslassen der Abschnitte 4a, 4b eingenommene Lage definiert eine Klemmlage, in welcher die Hülse in der Haltevorrichtung nicht verschiebbar ist.

## Patentansprüche

1. Vorrichtung (1) zum Halten einer eine Oberfläche, insbesondere die Bauchdecke, eines menschlichen oder tierischen Körpers durchdringenden Hülse (2) auf der Oberfläche, insbesondere einer Hülse (2) für die Endoskopie,
welche Vorrichtung (1) mit einer durchgehenden, vorzugsweise zylindrischen Längsöffnung (3) versehen ist, in welcher die Hülse (2) verschiebbar einsetzbar ist,
dadurch gekennzeichnet, dass die Vorrichtung (1) wenigstens zwei Abschnitte (4a, 4b) aufweist,
die je einen Teil (3a, 3b) der Längsöffnung (3) aufweisen, und die derart federnd miteinander verbunden sind, dass sie sich in einer Klemmlage (K) an der eingesetzten Hülse verspannen und dass sie gegen die Federkraft in eine Verschiebelage (V) bringbar sind, in der die Hülse in Längsrichtung (L) verschiebbar ist.

2. Vorrichtung nach Anspruch 1, dadurch gekennzeichnet, dass die Abschnitte je eine Längsöffnung (3a, 3b) aufweisen und dass in der Klemmlage (K) die gegenseitige Lage der Abschnitte (4a, 4b) derart gewählt ist, dass die Längsrichtungen (La, Lb) der Längsöffnungen (3a, 3b) der Abschnitte (4a, 4b) in einem Winkel (α) zueinander stehen, sodass eine in die Längsöffnung (3) eingesetzte Hülse sich verklemmt und
dass in der Verschiebelage (V) die Längsrichtungen (La, Lb) etwa parallel zueinander verlaufen, so dass eine in die Längsöffnung (3) eingesetzte Hülse verschiebbar ist.

3. Vorrichtung nach einem der Ansprüche 1 oder 2, dadurch gekennzeichnet, dass die Vorrichtung (1) einstückig, insbesondere aus einem sterilisierbaren Kunststoffmaterial, ausgebildet ist.

4. Vorrichtung nach Anspruch 3, dadurch gekennzeichnet, dass die federnde Verbindung als ein einstückig mit den Abschnitten 4a, 4b gebildetes Biegegelenk (5) ausgeformt ist, wobei die Abschnitte (4a, 4b) durch einen etwa senkrecht zur Längsrichtung (7a, 7b) der Längsöffnungen (3a, 3b) verlaufenden Spalt (6) voneinander getrennt sind.

5. Vorrichtung nach einem der Ansprüche 1 bis 4, dadurch gekennzeichnet, dass die Abschnitte (4a, 4b) mit je wenigstens einer vorstehenden Grifflasche (7a, 7b) versehen sind, mittels welcher die Abschnitte (4a, 4b) gegeneinander zusammendrückbar sind.

6. Vorrichtung nach einem der Ansprüche 1 bis 5, dadurch gekennzeichnet, dass die Vorrichtung (1) einen etwa konischen Dichtteil (8) aufweist.

7. Verfahren zum Herstellen einer Vorrichtung nach einem der Ansprüche 1 bis 6, gekennzeichnet durch die Schritte
- Bereitstellen eines Rohlings mit zwei federnd miteinander verbundenen Abschnitten (4a, 4b)
- Spannen, insbesondere Zusammendrücken der Abschnitte gegen die federnde Verbindung in eine Verschiebelage (V)
- Anbringen einer die beiden Abschnitte (4a, 4b) durchdringenden Längsöffnung (3), insbesondere einer Bohrung.

8. Verfahren nach Anspruch 7, dadurch gekennzeichnet, dass die Abschnitte (4a, 4b) durch Anbringen, insbesondere Einfräsen eines Spaltes (6) im Rohling voneinander getrennt werden.

9. Verfahren nach einem der Ansprüche 7 oder 8, dadurch gekennzeichnet, dass die Abschnitte (4a, 4b) nach Anbringen der Längsöffnung (3) in der Verschiebelage (V) gehalten werden und dass anschliessend eine Hülse (2) in die Längsöffnung (3) eingesetzt wird.

10. Verwendung einer Vorrichtung nach einem der Ansprüche 1 bis 6, zum Halten einer die Oberfläche, insbesondere die Bauchdecke eines menschlichen oder tierischen Körpers durchdringenden Hülse auf dieser Oberfläche.
